# EUROPÄISCHE PATENTANMELDUNG

(11) **EP 1 797 813 A1**
(43) Veröffentlichungstag der Anmeldung: **20.06.2007**
(21) Anmeldenummer: 06125351.4
(22) Anmeldetag: 04.12.2006
(51) Int. Cl.: A61B 1/05, A61B 5/00, A61B 5/107, G01B 11/24, G01N 21/954, G02B 23/24

(54) **Optische Messvorrichtung zum Vermessen eines Hohlraums**

(30) Priorität: 13.12.2005 DE 102005059550
(71) Anmelder: SIEMENS AKTIENGESELLSCHAFT, 80333 München (DE)
(72) Erfinder: Schick, Anton, 84149, Velden (DE)

(57) **Zusammenfassung**

Es wird eine optische Messvorrichtung 500 zum Vermessen der Innenwand eines in einem Objekt 595 ausgebildeten Hohlraums beschrieben. Die Messvorrichtung 500 umfasst einen konfokalen Abstandssensor, bei dem der Strahlengang des Beleuchtungs- und des Messlichts mittels eines drehbar gelagerten Reflektors 540 umgelenkt wird. Auf diese Weise kann durch ein Einführen zumindest eines objektseitigen Endes der Messvorrichtung 500 in den zu vermessenden Hohlraum eine umlaufende Seitenwand des Hohlraums vermessen werden. Der Reflektor 540 wird um eine Drehachse rotiert, welche bevorzugt mit der Längsachse der optischen Messvorrichtung 500 zusammenfällt. Auf diese Weise erfolgt ein Abtasten der Innenwand entlang einer die Drehachse umlaufenden Linie. Die optische Messvorrichtung 500 kann auch mit zwei konfokalen Abstandssensoren ausgestattet sein, wobei am objektseitigen Ende der Messvorrichtung 500 eine mehrere optische Komponenten 540, 535a, 535b aufweisende Doppeloptik vorgesehen ist, mittels welcher die Beleuchtungsstrahlen der beiden konfokalen Abstandssensoren bevorzugt diametral nach außen auf einander gegenüberliegende Abtastpunkte an der Innenwand des zu vermessenden Hohlraums fokussiert werden.

## Beschreibung

Die Erfindung betrifft eine optische Messvorrichtung zum Vermessen der Innenwand eines in einem Objekt ausgebildeten Hohlraums nach dem konfokalen Abbildungsprinzip. Die Erfindung betrifft insbesondere eine derartige optische Messvorrichtung zur Vermessung eines zylindrischen Bohrlochs und/oder zur Vermessung des Ohrkanals eines menschlichen oder eines tierischen Lebewesens.

Bei der Herstellung von Flugzeugen ist eine Vielzahl von Einzelverbindungen erforderlich, um unterschiedliche Komponenten eines Flugzeugs miteinander zu verbinden. So werden beispielsweise Tragflächen mittels einer Vielzahl von NietVerbindungen an dem Rumpf des Flugzeugs befestigt. Da aus Sicherheitsgründen die Präzision der Verbindungen äußerst hoch sein muss, müssen die für eine Nietverbindung erforderlichen Bohrungen mit hoher Genauigkeit durchgeführt werden, damit stets eine optimal passende Niete für das jeweilige Bohrloch verwendet werden kann. Aufgrund der Größe der miteinander zu verbindenden Einzelteile werden die Bohrlöcher typischerweise mit mobilen Bohrmaschinen gebohrt, die zum Bohren an das große Einzelteil herangeführt werden. Dabei ist die Präzision der gebohrten Löcher jedoch typischerweise etwas geringer als die Präzision von Bohrlöchern, die mit stationären Präzisionsbohrmaschinen gebohrt werden. Aus diesem Grund ist bei Flugzeugbau eine genaue Vermessung des jeweils gebohrten Loches erforderlich, um auch tatsächlich die passende Niete für das Loch zu verwenden. Die Vermessung der zylindrischen Bohrlöcher erfolgt heutzutage mittels kapazitiven Messverfahren, welche jedoch sehr unzuverlässig sind und eine geringe Orts- bzw. Tiefenauflösung aufweisen.

Zur Vermessung von zylindrischen Hohlräumen sind auch spezielle Koordinaten-Messmaschinen bekannt, welche eine genaue Vermessung von zylindrischen Bohrlöchern ermöglichen. Die Messgeschwindigkeit derartiger Messmaschinen ist jedoch üblicherweise sehr gering. Außerdem können derartige Koordinaten-Messmaschinen in der Regel nur stationär in speziellen Messlabors eingesetzt werden, so dass das jeweils zu vermessende Objekt zu der Messmaschine gebracht werden muss. Ein Einsatz in der Flugzeugindustrie bei der Vermessung von Hohlräumen in besonders großen Objekten, beispielsweise in dem Rumpf eines Flugzeuges oder in der Tragfläche eines Flugzeuges, scheidet demzufolge aus.

Auch bei dreidimensionalen Vermessungen von Hohlräumen des menschlichen Körpers ist es wünschenswert eine Messung sowohl mit einer hohen Präzision als auch mit einer hohen Messgenauigkeit durchzuführen. Derartige Messungen sind beispielsweise bei der Anpassung eines Hörgerätes in einen menschlichen Ohrkanal erforderlich. Zum Anpassen eines Hörgerätes wird derzeit von dem Ohrkanal ein Formabdruck hergestellt, welcher mit einer eigenen dreidimensional auflösenden optischen Messvorrichtung vermessen wird. Anhand der gewonnenen dreidimensionalen Daten des Formabdrucks kann ein für jeden Patienten individuell angepasstes Hörgerät bereitgestellt werden. Die Methode des Formabdrucks hat jedoch den Nachteil, dass der Ohrkanal nicht direkt, sondern lediglich indirekt durch eine Vermessung des Formabdrucks vermessen wird. Dies führt zu Ungenauigkeiten bei den hergestellten Hörgeräten und zu einem entsprechend reduzierten Tragekomfort.

Zur hochgenauen dreidimensionalen Vermessung von kleinen Objekten ist aus der DE 196 084 68 C2 ein optischer Abstandssensor bekannt, welcher auf dem konfokalen optischen Abbildungsprinzip beruht. Ein derartiger Abstandssensor, der beispielsweise in Figur 7 der genannten Druckschrift dargestellt ist, umfasst eine punktförmige Lichtquelle und einen punktförmigen Empfänger. Die punktförmige Lichtquelle wird auf einer Oberfläche eines Messobjekts abgebildet. Der punktförmige Empfänger ist im bildseitigen Messbereich konfokal zu der punktförmigen Lichtquelle angeordnet. Der Abstandssensor zeichnet sich objektseitig durch eine koaxiale Führung von Beleuchtungs- und Messlicht aus. Die optische Wegstrecke zwischen dem punktförmigen Empfänger und dem Messobjekt ist durch den Einsatz eines schwingenden Spiegelsystems variierbar. Durch eine Bestimmung der maximalen, von dem Empfänger gemessenen Lichtintensität als Funktion der Position des schwingenden Spiegelsystems kann die Höhenlage des jeweiligen Messpunktes auf der Oberfläche des Messobjekts bestimmt werden.

Aus der DE 101 258 85 B4 ist eine Sensorvorrichtung zur schnellen optischen Abstandsmessung nach dem konfokalen Abbildungsprinzip bekannt, bei der sowohl die punktförmige Lichtquelle als auch der punktförmige Lichtempfänger mittels eines Endes eines optischen Lichtwellenleiters realisiert sind. Die Sensorvorrichtung ist in zwei Module, einem optischen Modul und einem elektronischen Modul, aufgebaut. Damit kann das optische Modul, welches für einen Messvorgang in die Nähe des vermessenden Objekts gebracht werden muss, in einer kompakten Bauform realisiert werden. Das Elektronik-Modul ist über den Lichtwellenleiter mit dem Optikmodul verbunden, so dass sich bei einer entsprechenden Länge des Lichtwellenleiters das Elektronikmodul in großem Abstand von dem zu vermessenden Objekt befinden kann.

Aus der EP 1 398 597 A1 ist ein konfokaler Abstandssensor bekannt, der anstelle eines beweglichen Reflektors bzw. einer beweglich gelagerten Optik eine Abbildungsoptik aufweist, welche infolge einer chromatischen Abberation unterschiedliche Spektralanteile eines bevorzugt weißen Beleuchtungslichts in unterschiedlichen Abständen von der Abbildungsoptik fokussiert. Durch eine entsprechende Farbanalyse des von einer zu vermessenden Objektoberfläche zurück gestreuten Lichts kann der Abstand zwischen der zu vermessenden Oberfläche und der dem Abstandssensor ermittelt werden.

Der Erfindung liegt die Aufgabe zugrunde, eine optische Messvorrichtung zum Vermessen der Innenwand eines in einem Objekt ausgebildeten Hohlraums zu schaffen, welche Messvorrichtung eine schnelle und zugleich präzise Vermessung ermöglicht.

Diese Aufgabe wird gelöst durch eine optische Messvorrichtung mit den Merkmalen des unabhängigen Anspruchs 1. Die optische Messvorrichtung ist zur Vermessung der Innenwand eines in einem Objekt ausgebildeten Hohlraums und insbesondere zur Vermessung eines zylindrischen Bohrlochs und/oder zur Vermessung des Ohrkanals eines menschlichen oder tierischen Lebewesens geeignet. Die erfindungsgemäße optische Messvorrichtung umfasst ein punktförmiges optisches Sendeelement, eingerichtet zum Aussenden von Beleuchtungslicht, eine Abbildungsoptik zum Fokussieren des Beleuchtungslichts in einem Objektbereich und ein punktförmiges optisches Empfangselement, welches in Bezug auf die Abbildungsoptik konfokal zu dem optischen Sendeelement angeordnet ist und welches zum Empfang von Messlicht eingerichtet ist, das von der Innenwand des zu vermessenden Hohlraums zumindest teilweise zurückgestreut und über die Abbildungsoptik auf das Empfangselement gelenkt wird. Die optische Messvorrichtung umfasst ferner eine Auswerteeinheit, welche mit dem Empfangselement gekoppelt ist und welche derart eingerichtet ist, dass eine maximale Intensität an Messlicht erfassbar ist. Außerdem umfasst die erfindungsgemäße optische Messvorrichtung einen Reflektor, welcher in dem Strahlengang des Beleuchtungslichts und des Messlichts angeordnet ist. Der Reflektor ist um eine Drehachse drehbar, so dass durch das Beleuchtungslicht die Innenwand des zu vermessenden Hohlraums entlang einer die Drehachse umlaufenden Linie abtastbar ist.

Der Erfindung liegt die Erkenntnis zugrunde, dass durch ein Abknicken des Strahlengangs des Beleuchtungslichts bzw. des Messlichts eine Oberflächenvermessung auch dann möglich ist, wenn die Oberfläche die Innenwand eines zu vermessenden Hohlraums ist, in dem der drehende Reflektor während der Hohlraumvermessung eingebracht ist. Bevorzugt ist der Reflektor relativ zu dem auftreffenden Beleuchtungslicht derart geneigt, dass das Beleuchtungslicht in einen Strahlengang gelenkt wird, der senkrecht zu der Drehachse verläuft. Dies bedeutet, dass der Reflektor in einem 45°-Winkel relativ zur Drehachse bzw. zu dem Strahlengang des auf den Reflektor auftreffenden Beleuchtungslichts geneigt ist. Das Beleuchtungslicht wird somit senkrecht zu der Drehachse an die Innenwand des Hohlraums gelenkt, wobei der Fokus des Beleuchtungslichts eine um die Drehachse umlaufende Kreislinie beschreibt.

Es wird darauf hingewiesen, dass die punktförmigen Elemente, das optische Sendeelement und das optische Empfangselement, auch durch die Kombination eines flächigen optischen bzw. optoelektronischen Elements mit einer davor angeordneten Lochblende realisiert werden können.

Die beschriebene optische Messvorrichtung hat gegenüber bekannten optischen Messmaschinen den Vorteil, dass die Messvorrichtung insbesondere senkrecht zur Drehachse in einer sehr kompakten, d.h. sehr schlanken Bauweise realisiert werden kann. Somit muss lediglich ein Teil der optischen Messvorrichtung, nämlich der drehbar gelagerte Reflektor, zur Vermessung der Innenwand eines Hohlraums in diesen Hohlraum eingebracht werden. Die übrigen Komponenten der optischen Messvorrichtung müssen für eine sowohl zügige als auch präzise Vermessung der Innenwand nicht in den Hohlraum eingebracht werden, so dass ein objektseitiger Teil der optischen Messvorrichtung innerhalb einer kompakten Bauform realisiert werden kann. Dies schafft die Möglichkeit, dass die optische Messvorrichtung als mobiles Gerät sowohl zur Vermessung von zylindrischen Bohrlöchern als auch zur Vermessung des Ohrkanals eines menschlichen oder tierischen Lebewesens eingesetzt werden kann.

Mittels geeigneter Bildverarbeitungsalgorithmen kann die Messgenauigkeit der optischen Messvorrichtung weiter erhöht werden.

Gemäß Anspruch 2 weist die optische Messvorrichtung zusätzlich ein Gehäuse und einen optischen Messkopf auf, welcher relativ zu dem Gehäuse entlang einer Verschieberichtung verschiebbar gelagert ist. Dabei sind das Sendeelement, die Abbildungsoptik, das Empfangselement und der Reflektor dem optischen Messkopf zugeordnet. Die Anordnung der genannten Komponenten der optischen Messvorrichtung in einem verschiebbar gelagerten Messkopf hat den Vorteil, dass der Hohlraum nicht nur entlang einer in einer bestimmten Tiefe des Hohlraums befindlichen Kreislinie, sondern entlang einer Vielzahl von verschiedenen Kreislinien, die sich in verschiedenen Tiefen des Hohlraums befinden, vermessen werden kann. Bevorzugt fällt die Verschiebeachse mit der Drehachse des drehbar gelagerten Reflektors zusammen. Dies ermöglicht auf einfache Weise eine vollständige Vermessung der Innenwand in verschiedenen Tiefen des Hohlraumes.

Gemäß Anspruch 3 weist die optische Messvorrichtung zusätzlich einen Antrieb zum axialen Verschieben des Messkopfes und ein Wegmeßsystem zur Erfassung der Verschiebeposition des Messkopfes relativ zu dem Gehäuse auf. Dies ermöglicht auf vorteilhafte Weise eine besonders präzise axiale Positionierung des Messkopfes und daraus resultierend eine besonders genaue vollständige Vermessung der Innenwandstruktur des zu vermessenden Hohlraums.

Gemäß Anspruch 4 umfasst das Gehäuse Adaptionselemente zum definierten Anlegen der optischen Messvorrichtung an das Objekt. Adaptionselemente können beispielsweise Saugnäpfe oder jede andere Art von mechanischen Koppelelementen sein, die während eines Messvorgangs eine präzise Justierung der optischen Messvorrichtung relativ zu dem zu vermessenden Hohlraum sicherstellen.

Bei der Vermessung eines menschlichen Ohrkanals zur Zwecke der Anpassung eines Hörgerätes ermöglicht die beschriebene Messvorrichtung im Gegensatz zu bekannten Abdruckverfahren auch eine genaue Vermessung des Ohrkanals in tieferen Bereichen. Dadurch können Hörgeräte auch für einen Einsatz in tieferen Bereichen des Ohrkanals genau gefertigt werden, so dass die entsprechenden Hörgeräte besser an das jeweilige Trommelfell angepasst werden können. Dadurch kann im Vergleich zu einem weiter außen im Ohrkanal befindlichen Hörgerät ein deutlich höherer Wirkungsgrad und damit ein besseres Hörvermögen des jeweiligen Patienten erreicht werden.

Gemäß Anspruch 5 umfasst die optische Messvorrichtung zusätzlich einen Drehantrieb zum Drehen des Reflektors um die Drehachse und einen Drehwinkeldetektor zum Erfassen des aktuellen Drehwinkels des Reflektors. Der Drehwinkeldetektor kann bevorzugt mit dem Drehantrieb über eine Regelungsschleife gekoppelt sein, so dass eine genau definierte Drehbewegung des Reflektors realisiert werden kann. Auf diese Weise können die jeweiligen Abtastpunkte der optischen Messvorrichtung präzise bestimmt werden, so dass bezogen auf den jeweiligen Abtastpunkt eine besonders hohe Genauigkeit der optischen Vermessung gewährleistet werden kann.

Im Gegensatz zu dreidimensionalen optischen Sensoren, welche nach dem Prinzip der Triangulation Höhendaten ermitteln, sind bei den hier beschriebenen Messvorrichtungen keine unterschiedlichen Beleuchtungs- und Beobachtungsrichtungen zur Ermittlung von Abstandswerten erforderlich. Somit können die Messvorrichtungen in kompakter Bauweise in Form von so genannten Handheld-Systemen aufgebaut werden. Dabei ist es vorteilhaft, dass zur Sicherstellung der Genauigkeit eine Vorzentrierung des jeweiligen Handheld-Systems relativ zu den zu vermessenden Hohlräumen, insbesondere zu zylinderförmigen Hohlräumen, erfolgt. Dies kann durch mechanische Zentrierbacken erfolgen, die nach der Zentrierung und Fixierung des Handheld-Systems gegebenenfalls entfernt werden können.

Gemäß Anspruch 6 sind das optische Sendeelement und/oder das optische Empfangselement durch das Ende eines Lichtwellenleiters realisiert. Das Sendeelement und das Empfangselement können somit auf einfache Weise auch an derselben Position angeordnet sein, so dass automatisch stets eine perfekt konfokale Anordnung zwischen Sendeelement und Empfangselement gewährleistet ist. Die optische Messvorrichtung lässt sich somit ohne großen Justieraufwand herstellen.

Um das Sendeelement mit einer Lichtquelle und das Empfangselement mit einem Lichtdetektor optisch zu koppeln, ist das andere Ende des Lichtellenleiters beispielsweise in zwei Teilenden aufgespalten oder mit einem Strahlteiler optisch gekoppelt. In beiden Fällen ist darauf zu achten, dass an dem anderen Ende des Lichtwellenleiters ein optischer Kanal mit der Lichtquelle und der andere optische Kanal mit dem Lichtdetektor optisch gekoppelt ist.

Die Verwendung eines Lichtwellenleiters hat jedoch auch noch den Vorteil, dass optoelektronische Komponenten, wie beispielsweise eine als Lichtquelle dienende Leuchtdiode oder eine als Lichtdetektor dienende Photodiode, räumlich von der Optik bzw. dem rotierenden Reflektor getrennt angeordnet sein können, so dass das objektseitige Ende der optischen Messvorrichtung in einer besonders kompakten Bauform realisiert werden kann.

Als Lichtquelle eignen sich insbesondere Leuchtdioden oder Halbleiterlaser, welche bei geringem Strombedarf eine hohe Lichtausbeute gewährleisten. Als Lichtdetektor eignen sich beispielsweise Photodioden, welche gegebenenfalls in Verbindung mit einer davor angeordneten Blende verwendet werden können und welche eine hohe Lichtempfindlichkeit aufweisen. Zusätzlich haben derartige optoelektronische Komponenten den Vorteil, dass sie preiswerte optoelektronische Komponenten sind.

Anspruch 7 beschreibt eine bevorzugte Ausführungsform der Erfindung, bei der im Strahlengang zwischen Sendeelement und Objektbereich ein dispersives optisches Element angeordnet ist und bei der das Empfangselement ein spektrales Auflösungsvermögen aufweist, so dass von der Auswerteeinheit die maximale Intensität an Messlicht, die auf das Empfangselement trifft, in Abhängigkeit von der Wellenlänge des Messlichts erfassbar ist.

Bei dieser Ausführungsform wird also eine wellenlängenabhängige räumliche Aufspaltung der Fokuslagen erzeugt. Diese Aufspaltung ist dann besonders deutlich, wenn weißes Beleuchtungslicht oder zumindest ein Beleuchtungslicht mit einem weiten Farbspektrum verwendet wird. Die konfokale Messung erfolgt dann durch eine einfache Spektralanalyse des zumindest teilweise an der Innenwand des zu vermessenden Hohlraums zurück gestreuten Messlichts. Die Messgenauigkeit ist dabei dann besonders hoch, wenn die Fokuslagen der einzelnen Spektralanteile des Beleuchtungslichts nahe beieinander liegen.

Das Empfangselement kann beispielsweise ein spektral auflösender Punktdetektor sein, welcher durch einen spektral auflösenden Flächendetektor in Verbindung mit einer davor angeordneten Lochblende mit einem ungefähren Durchmesser von 20µm bis 100µm realisiert werden kann. Alternativ kann auch hinter der Lochblende ein Spektrometer angeordnet sein, welches die einzelnen Farbanteile in leicht unterschiedliche Richtungen lenkt. Die wellenlängenabhängige Intensitätsmessung erfolgt dann in bekannter Weise durch einen ortsauflösenden Lichtdetektor.

Die Verwendung eines dispersiven optischen Elements hat den Vorteil, dass die optische Messvorrichtung mit Ausnahme des drehbaren Reflektors keine weiteren Komponenten erfordert, welche zur optischen Vermessung eine mechanische Bewegung erfordern. Somit kann die optische Messvorrichtung nicht nur in einer kompakten Bauform, sondern auch in einer mechanisch sehr robusten Bauweise realisiert werden, so dass auch bei mechanischen Erschütterungen der optischen Messvorrichtung die Ausfallwahrscheinlichkeit entsprechend gering ist. Damit ist die optische Messvorrichtung infolge ihrer großen Robustheit vielfältig, also auch in einem mechanisch sehr rauen Fertigungsumfeld einsetzbar.

Gemäß der vorteilhaften Ausführungsform nach Anspruch 8 stellt die Abbildungsoptik das dispersive Element dar. Ferner ist zwischen der Abbildungsoptik und dem Reflektor ein Lichttransportelement vorgesehen, welches gemeinsam mit dem Reflektor um die Drehachse drehbar gelagert ist. Das Lichttransportelement kann aus einem beliebigen Material hergestellt sein, welches optisch transparent ist. Dabei ist lediglich erforderlich, dass sich das von der Abbildungsoptik gebrochene Licht derart ausbreiten kann, dass die optische Abbildung bzw. die Fokussierung durch die Abbildungsoptik nicht oder in einer genau definierten Weise beeinträchtigt wird. Das Lichttransportelement, welches beispielsweise ein Stab ist, kann auch als mechanische Halterung für den um die Drehachse rotierenden Reflektor dienen.

Anspruch 9 beschreibt eine weitere bevorzugte Ausführungsform der Erfindung, bei der die optische Messvorrichtung zusätzlich Mittel zur Variation der Fokuslage aufweist. Das oder die Mittel zur Variation der Fokuslage kann oder können beispielsweise ein verschiebbar gelagerter Spiegel oder ein Retroreflektor sein. Der Tiefenbereich, in dem die Fokuslage variiert werden kann, definiert dabei den Messbereich der optischen Messvorrichtung.

Gemäß Anspruch 10 umfasst das Mittel zur Variation der Fokuslage eine Lagerung, welche eine Verschiebung der Abbildungsoptik entlang einer Verschieberichtung ermöglicht. Dabei verläuft die Verschieberichtung parallel zu der optischen Achse der Abbildungsoptik. Durch die Verschiebung der Abbildungsoptik kann auf besonders einfache Art und Weise, d.h. insbesondere ohne zusätzliche weitere Reflektoren, eine zeitliche Variation der Fokuslage erreicht werden.

Anspruch 11 beschreibt eine weitere bevorzugte Ausführungsform der vorliegenden Erfindung, bei der die Abbildungsoptik zwischen Reflektor und Objektbereich angeordnet und gemeinsam mit dem Reflektor um die Drehachse drehbar ist. Dabei wird die Abbildungsoptik mittels eines Federelements in einer in Bezug auf die Drehachse radialen Position gehalten. Dabei ist die radiale Position unter dem Einfluss einer Zentrifugalkraft variierbar, die bei einer Drehung auf die Abbildungsoptik wirkt. Bei einer derartigen passiven Lagerung der Abbildungsoptik, bei der keine aktiven Stellglieder für eine Verschiebung der Abbildungsoptik erforderlich sind, hängt die Soll-Lage der Abbildungsoptik lediglich über das so genannte Hook'sche Gesetz von der Federkonstante des Federelements und von der Drehgeschwindigkeit, insbesondere der Winkelgeschwindigkeit, des Reflektors ab. Die Lagerung kann demzufolge auf einfache Weise innerhalb einer kompakten Bauform realisiert werden, so dass durch die optische Messvorrichtung auch Hohlräume mit einem sehr kleinen Innendurchmesser vermessen werden können.

Es wird darauf hingewiesen, dass anstelle der beweglichen Abbildungsoptik zusätzlich zu einer festen Abbildungsoptik auch ein weiterer Reflektor, beispielsweise ein Retroreflektor, mit dem Federelement mechanisch gekoppelt sein kann. Der weitere Reflektor muss dann derart passiv gelagert sein, sich ebenfalls die Fokuslage des Beleuchtungslichts in Abhängigkeit von der durch die Drehbewegung verursachten Zentrifugalkraft ändert.

Anspruch 12 beschreibt eine weitere Ausführungsform der vorliegenden Erfindung, bei der das Mittel zur Variation der Fokuslage ein aktives Stellglied ist. Dies hat den Vorteil, dass die Fokuslage unabhängig von der jeweiligen Winkelgeschwindigkeit eingestellt werden kann.

Gemäß Anspruch 13 weist das Mittel zur Variation der Fokuslage zusätzlich ein weiteres Wegmesssystem auf. Dies hat den Vorteil, dass die Fokuslage durch eine Regelung, welche eine zwischen dem Wegmesssystem und dem aktiven Stellglied geschaltete Regelungsschleife aufweist, besonders präzise variiert werden kann. Damit kann die Innenwand mit einer besonders großen Genauigkeit vermessen werden.

Anspruch 14 beschreibt eine weitere, besonders bevorzugte Ausführungsform der Erfindung. Demnach umfasst die optische Messvorrichtung zusätzlich (a) ein weiteres punktförmiges optisches Sendeelement, eingerichtet zum Aussenden von weiteren Beleuchtungslicht, (b) eine weitere Abbildungsoptik zum Fokussieren des weiteren Beleuchtungslichts in einen weiteren Objektbereich, (c) ein weiteres punktförmiges optisches Empfangselement, welches in Bezug auf die weitere Abbildungsoptik konfokal zu dem weiteren optischen Sendeelement angeordnet ist, welches zum Empfang von weiterem Messlicht eingerichtet ist, das von der Innenwand des zu vermessenden Hohlraums zumindest teilweise zurückgestreut und über die weitere Abbildungsoptik auf das weitere Empfangselement gelenkt wird, und welches ebenfalls mit der Auswerteeinheit gekoppelt ist, so dass eine maximale Intensität an weiterem Messlicht erfassbar ist, die auf das weitere Empfangselement trifft. Die optische Messvorrichtung umfasst ferner (d) einen weiteren Reflektor, welcher in den Strahlengang des weiteren Beleuchtungslichts und des weiteren Messlichts angeordnet ist. Dabei ist der weitere Reflektor winkelig zu dem Reflektor angeordnet und gemeinsam mit dem Reflektor um die Drehachse drehbar, so dass durch das weitere Beleuchtungslicht die Innenwand des zu vermessenden Hohlraums ebenfalls entlang einer die Drehachse umlaufende Linie abtastbar ist, wobei das Beleuchtungslicht und das weitere Beleuchtungslicht in Bezug auf die Drehachse in unterschiedliche Richtungen an die Innenwand des Hohlraums gelenkt werden.

Dieser Ausführungsvariante liegt die Erkenntnis zugrunde, dass die optische Messvorrichtung mit einem zweiten konfokalen Abstandssensor ausgestattet werden kann, welcher gleichzeitig mit dem ersten konfokalen Abstandssensor die Innenwand des zu vermessenden Hohlraums an einem anderen Messpunkt abtastet. Dies ermöglicht auf vorteilhafte Weise eine im Vergleich zu der Verwendung eines einzigen konfokalen Abstandssensor in der optischen Messvorrichtung eine um einen Faktor zwei schnellere Aufnahme der Höhendaten der zu vermessenden Innenwand.

Es wird darauf hingewiesen, dass unter dem Begriff Höhendaten sind in diesem Zusammenhang die Abstandswerte der jeweiligen Abtastpunkte auf der zu vermessenden Innenwand von der Drehachse zu verstehen sind.

Gemäß Anspruch 15 sind der Objektbereich und der weitere Objektbereich im Bezug auf die Drehachse diametral zueinander angeordnet. Dies bedeutet, dass die beiden Abtastbereiche zueinander um einen Winkel von 180° versetzt sind. Durch ein derartiges gleichzeitiges Vermessen des Hohlraums an diametral gegenüberliegenden Stellen kann eine gegebenenfalls vorhandene nicht zentrale Anordnung der Drehachse in Bezug auf den zu vermessenden Hohlraum durch eine entsprechende Datenauswertung korrigiert werden. Taumelfehler der rotierenden Drehachse können somit auf vorteilhafte Weise kompensiert werden.

Weitere Vorteile und Merkmale der vorliegenden Erfindung ergeben sich aus der folgenden beispielhaften Beschreibung derzeit bevorzugter Ausführungsformen. In der Zeichnung zeigen in schematischen Darstellungen:
- Figur 1: eine optische Messvorrichtung mit einer dispersiven Abbildungsoptik zur gleichzeitigen Erzeugung von spektral unterschiedlichen Fokuslagen,
- Figur 2: eine optische Messvorrichtung mit einer dispersiven Abbildungsoptik zur gleichzeitigen Abstandsmessung an zwei unterschiedlichen Abtastpunkten,
- Figur 3a: eine optische Messvorrichtung mit einer Abbildungsoptik, welche durch ein Kräftegleichgewicht zwischen einer Federkraft und einer Zentrifugalkraft in einer Verschiebeposition gehalten wird,
- Figur 3b: eine vergrößerte Darstellung der in Figur 3a dargestellten passiven Lagerung der Abbildungsoptik,
- Figur 4: eine optische Messvorrichtung zur Messung von zylindrischen Innenmantelflächen an einander diametral gegenüberliegenden Abtastpunkten mit aktiven Antrieben zur Variation der Fokuslage, wobei eine optische Kopplung zwischen Sendeelement bzw. Empfangselement mit einer Lichtquelle bzw. einem Lichtdetektor über eine Faseroptik erfolgt,
- Figur 5: eine Variante der in Figur 4 dargestellten Messvorrichtung, wobei eine optische Kopplung zwischen Sendeelement bzw. Empfangselement mit einer Lichtquelle bzw. einem Lichtdetektore über eine Freistrahloptik erfolgt, und
- Figur 6: eine vergrößerte Darstellung einer weiteren Variante der in Figur 4 dargestellten Messvorrichtung, wobei zwei Abtaststrahlen durch eine Aufspaltung eines von einem einzigen punktförmigen optischen Element generierten Beleuchtungslicht erzeugt werden, wobei das punktförmige optische Element durch das Ende eines Lichtwellenleiters realisiert ist.

An dieser Stelle bleibt anzumerken, dass sich in der Zeichnung die Bezugszeichen von gleichen oder voneinander entsprechenden Komponenten lediglich in ihrer ersten Ziffer und/oder durch einen angehängten Buchstaben unterscheiden.

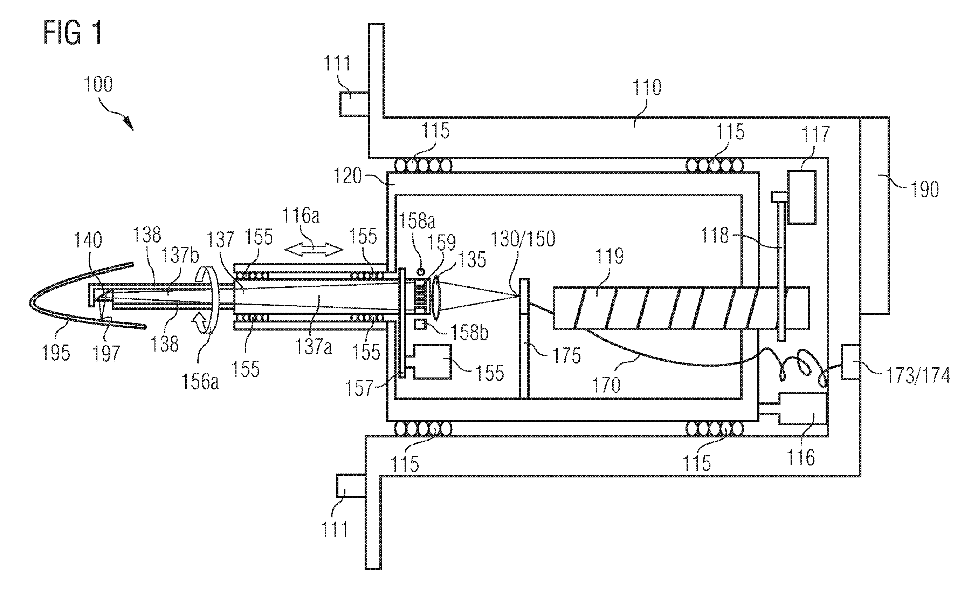

Figur 1 zeigt eine konfokale Messvorrichtung 100 mit einem so genannten chromatischen konfokalen Abstandssensor. Die hier dargestellte Messvorrichtung 100 dient der Vermessung des Gehörgangs einer Ohrmuschel 195. Dabei können räumliche Daten der Innenstruktur des Gehörgangs ermittelt werden, so dass ein passgenaues Hörgerät hergestellt werden kann.

Die Messvorrichtung 100 umfasst ein Gehäuse 110, an welchem Adaptionselemente 111 vorgesehen sind, welche ein definiertes Aufsetzen des Gehäuses 110 seitlich an dem nicht dargestellten Kopf eines Patienten ermöglichen. In dem Gehäuse 110 ist ein Messkopf 120 vorgesehen, in welchem der eigentliche chromatische konfokale Abstandssensor aufgebaut ist. Der Messkopf 120 ist relativ zu dem Gehäuse 110 axial verschiebbar gelagert, so dass mittels eines Axialantriebs 116 der Messkopf 120 entlang einer Verschieberichtung 116a relativ zu dem Gehäuse 110 positioniert werden kann. Um eine definierte axiale Verschiebung des Messkopfes 120 zu gewährleisten, sind mehrere Linearlager 115 vorgesehen, die zwischen einer Außenwand des Messkopfes 120 und einer Innenwand des Gehäuses 110 angeordnet sind. Um die genaue axiale Verschiebeposition des Messkopfes 120 relativ zu dem Gehäuse 110 zu bestimmten, ist ferner ein axiales Wegmesssystem 117 vorgesehen, welches einen Messfühler 118 aufweist, der die jeweilige Verschiebeposition des Messkopfes 120 an einem an dem Messkopf 120 angebrachten Maßstab 119 abgreift.

In dem Messkopf 120 befindet sich eine Punktlichtquelle 130, welche durch das Ende eines Lichtwellenleiters 170 realisiert ist. Das Ende des Lichtwellenleiters 170 ist mittels einer Halterung 175 in dem Messkopf 120 räumlich fixiert. Der Lichtwellenleiter 170 ist bevorzugt eine so genannte Multimode-Faser, so dass die effektive Querschnittsfläche der Punktlichtquelle 130 eine Kreisscheibe mit einem Durchmesser von lediglich einigen µm darstellt. Die Einspeisung von Licht in den Lichtwellenleiter 170 erfolgt mittels eines optoelektronischen Moduls, welches mit dem anderen Ende des Lichtwellenleiters 170 optisch gekoppelt ist und welches eine Weißlichtquelle 173 sowie einen spektral auflösenden Lichtdetektor 174 aufweist. Somit ist das zweite Ende des Lichtwellenleiters 130 sowohl mit der Weißlichtquelle 173 als auch mit der Lichtdetektor 174 optisch gekoppelt, so dass das erste Ende des Lichtwellenleiters 170 nicht nur die Punktlichtquelle 130, sondern auch einen Punktlichtdetektor 150 darstellt.

Im folgenden wird die Funktion des chromatischen konfokalen Abstandssensors erläutert: Von der Punktlichtquelle 130 emittiertes Beleuchtungslicht trifft auf eine Abbildungsoptik 135, welche eine hohe chromatische Aberration aufweist. Die Abbildungsoptik 135 ist an einem Glasstab 137 befestigt, welcher mittels mehrerer Drehlager 155 relativ zu dem Messkopf 120 drehbar gelagert ist. Der Glasstab 137 weist einen dicken Abschnitt 137a sowie einen dünnen Abschnitt 137b auf, wobei die stufenweise Verjüngung des Glasstabes 137 so gewählt ist, dass sich das von der Abbildungsoptik 135 fokussierte Beleuchtungslicht ohne seitliche Reflexionen an der Außenmantelfläche der beiden Glasstababschnitte 137a und 137b bis hin zu dem Austritt an einer Stirnseite des Abschnittes 137b frei ausbreiten kann. An dem dünnen Abschnitt 137b des Glasstabes 137 ist ein Schutzmantel 138 vorgesehen, welcher sich über das Ende des Glasstabes 137b hinaus erstreckt. Der Schutzmantel 138 dient gleichzeitig als Halterung für einen Reflektor 140. Das von der Punktlichtquelle 130 emittierte Beleuchtungslicht weist eine große spektrale Breite auf. Bevorzugt ist das Beleuchtungslicht weißes Licht. Das Beleuchtungslicht wird infolge der Dispersion der Abbildungsoptik 135 abhängig von dem jeweiligen spektralen Anteil unterschiedlich stark fokussiert, so dass nach einer Reflexion an dem Reflektor 140 für unterschiedliche Farbanteile unterschiedliche Fokuslagen 197 erzeugt werden.

Infolge der konfokalen Anordnung zwischen Punktlichtquelle 130 und Punktlichtdetektor 150, welche sich gemäß dem hier dargestellten Ausführungsbeispiel an derselben Stelle befinden, wird von dem an der Innenfläche der Ohrmuschel 195 zumindest teilweise zurück gestreuten Messlicht derjenige Farbanteil am stärksten von dem Punktlichtdetektor 150 detektiert, dessen zugehörige Fokuslage genau auf der Oberfläche der Innenwand der Ohrmuschel 195 liegt. Somit kann durch eine entsprechende Farbanalyse des zumindest teilweise zurück gestreuten und in den Lichtwellenleiter 170 eingekoppelten Messlichts der Abstand des jeweiligen Abtastpunktes von dem Abstandssensor bzw. von dem Reflektor 140 ermittelt werden. Zu diesem Zweck umfasst das optoelektronische Modul entweder einen spektral auflösenden Lichtdetektor 174 oder ein Spektrometer, welches vor dem Lichtdetektor 174 vorgesehen ist, die in diesem Fall ein ortsauflösendes Photodiodenarray ist.

Um die Innenwand der Ohrmuschel 195 vollständig zu vermessen, ist der Glasstab 137 mittels mehrerer Drehlager 155 in dem Messkopf 120 drehbar gelagert. Die Drehung des Glasstabes 137 erfolgt mittels eines Drehantriebs 156, welcher über einen Riemen 157 mit dem Glasstab 137 gekoppelt ist. Bei einer entsprechenden Ansteuerung des Drehantriebs 156, welche durch ein Elektronikmodul 190 erfolgt, kann der Glasstab 137 und mit dem Glasstab 137 auch der Reflektor 140 beispielsweise entgegen des Uhrzeigersinns in einer Drehrichtung 156a gedreht werden. Das Elektronikmodul umfasst neben der Ansteuerelektronik für den Drehantrieb 156 auch die Steuer-Regel- und Messelektronik für sämtliche elektrischen und optoelektronischen Komponenten der konfokalen Messvorrichtung 100.

Um jeweils eine genaue Kenntnis des aktuellen Drehwinkels des Reflektors 140 zu haben, ist an dem vorderen Ende des Glasstabes 137a eine Markierung 159 ausgebildet. Diese Markierung ist mittels einer Messeinrichtung detektierbar, welche eine Leuchtdiode 158a und eine Photodiode 158b enthält. Die Markierung 159 umfasst mehrere optisch nicht transparente Striche, so dass durch ein Zählen der Lichtzyklen, die bei einer Drehung des Glasstabes 137 zu einem wiederholten Abblocken des von der Leuchtdiode 158a emittierten und von der Photodiode 158b detektierten Lichts führen, der aktuelle Drehwinkelbestimmt werden kann.

Es wird darauf hingewiesen, dass mittels der Drehwinkel-Messeinrichtung nicht nur der aktuelle Drehwinkel des Glasstabes 137 exakt vermessen, sondern dass eine präzise Regelung der Drehgeschwindigkeit des Glasstabes 137 erreicht werden kann. Dies kann beispielsweise dadurch erreicht werden, dass die Photodiode 158b mit dem Drehantrieb 156 mittels einer Regelungsschleife gekoppelt ist. Die Regelung der Drehgeschwindigkeit kann ebenfalls über das Elektronikmodul 190 erfolgen.

Eine vollständige Vermessung der Innenstruktur der Ohrmuschel 195 kann beispielsweise dadurch erfolgen, dass der vordere Teil des Messkopfes 120 tief in den Ohrkanal eingeführt wird und bei einer konstanten Drehung des Messkopfes 120 dieser langsam aus dem Ohrkanal herausgezogen wird. Eine Vermessung des Ohrkanals, bei der der Messkopf 120 lediglich nach Außen hin verschoben wird, hat den Vorteil, dass der Ohrkanal 195 durch den an der Innenwand des Ohrkanals 195 reibenden Messkopf 120 lediglich in geringer und zudem definierter Art und Weise deformiert wird. Im Vergleich zu dem Fall, bei dem der Messkopf 120 nach innen in tiefere Bereiche des Ohrkanals 195 eingeführt wird und somit bei einer Reibung das Gewebe des Ohrkanals 195 zusammen schiebt, verursacht ein langsames Herausziehen des an der Innenwand des Ohrkanals 195 reibenden Messkopfes 120 deutlich geringere Deformationen des zu vermessenden Ohrkanals 195.

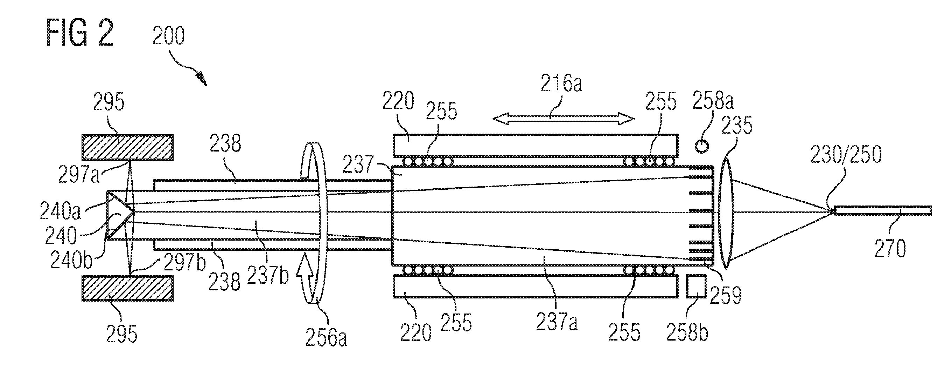

Figur 2 zeigt den vorderen, d.h. den objektseitigen Teil einer chromatischen konfokalen Messvorrichtung 200, welcher sich von der konfokalen Messvorrichtung 100 lediglich dadurch unterscheidet, dass das Beleuchtungslicht auf zwei einander diametral gegenüberliegenden Fokusbereichen 297a und 297b fokussiert wird. Auf diese Weise kann der Innenraum eines Objekts 295, welcher bevorzugt ein zylindrischer Hohlraum ist, gleichzeitig an jeweils zwei Abtastpunkten vermessen werden. Die konfokale Messvorrichtung 200 umfasst neben einem nicht dargestellten Gehäuse einen in Figur 2 teilweise dargestellten Messkopf 200, welcher entlang einer Verschieberichtung 216a relativ zu dem nicht dargestellten Gehäuse axial verschiebbar ist. Die konfokale Messvorrichtung 200 umfasst ferner eine Punktlichtquelle 230 sowie einen Punktlichtdetektor 250, welche durch ein Ende eines Lichtwellenleiters 270 realisiert sind.

Die als Chromat wirkende Abbildungsoptik 235, der Glasstab 237 mit dem dicken Abschnitt 237a und dem dünnen Abschnitt 237b sowie der Schutzmantel 238 wurden bereits zuvor anhand von Figur 1 beschrieben und werden deshalb an dieser Stelle nicht noch einmal im Detail erläutert. Dasselbe gilt für die Drehlager 255, welche eine Rotation des Glasstabes 237 entlang der Drehrichtung 256a erlauben, sowie für die Drehwinkel-Messeinrichtung, welche die Leuchtdiode 258a, die Photodiode 258b sowie die Markierung 259 auf dem Glasstab 237 umfasst.

Wie aus Figur 2 ersichtlich, ist der drehbare Reflektor ein Prisma 240, welches zwei Begrenzungsflächen 240a und 240b aufweist. Diese Begrenzungsflächen 240a und 240b wirken als drehbare Reflektoren. Der von der Abbildungsoptik 235 fokussierte Beleuchtungsstrahl trifft auf die Spitze des Prismas 240, so dass ein Teil des Beleuchtungslichts in Richtung des Fokusbereiches 297a und ein anderer Teil des Beleuchtungslichts in Richtung des Fokusbereiches 237b gelenkt wird. Dort erfolgt an der Innenwand des Objekts 295 in analoger Weise eine zumindest teilweise Reflexion, so dass aufgrund der konfokalen Anordnung von Punktlichtwelle 230 und Punktlichtdetektor 250 bevorzugt derjenige Spektralbereich in den Lichtquellen 270 eingekoppelt ist, dessen Fokuslage genau mit der inneren Oberfläche des in dem Objekt 295 ausgebildeten Hohlraums zusammenfällt.

Es wird darauf hingewiesen, dass bei dem hier dargestellten Ausführungsbeispiel trotz der beiden diametral gegenüber liegenden Abtastbereiche 297a und 297b lediglich eine Punktlichtquelle 230 bzw. ein Punktlichtdetektor 250 vorgesehen ist. Dies bedeutet, dass bei der Auswertung der Messdaten zwischen den beiden Abtastbereichen 297a und 297b nicht unterschieden werden kann. Demzufolge sollte für eine präzise Vermessung des in dem Objekt 295 ausgebildeten zylindrischen Hohlraums die Messvorrichtung 200 genau mittig in das zu vermessende zylindrische Loch eingeführt werden. Im Falle einer nichtzentrischen Vermessung des Loches entsteht nämlich ein unscharfes Messsignal, weil von dem einen Fokusbereich 297a bevorzugt Licht in einem ersten Spektralbereich und von dem zweiten Fokusbereich 297b bevorzugt Licht mit einem zweiten, von dem ersten Spektralbereich unterschiedlichen Spektralbereich in den Lichtwellenleiter 270 eingekoppelt wird.

Ein derartiges vermeintlich uneinheitliches Messsignal, bei dem zwei maximale Intensitäten in voneinander unterschiedlichen Spektralbereichen gemessen werden, kann jedoch auch dazu verwendet werden, die Exzentrizität, d. h. die Abweichung der Drehachse der Messvorrichtung 230 von der Mittelachse des zylindrischen Loches in dem Objekt 295 zu ermitteln. Diese Ermittlung kann beispielsweise dadurch erfolgen, dass neben der Messwerte für die absolute Lage der beiden Intensitätsmaxima auf der Frequenzachse auch der Frequenzabstand der beiden Intensitätsmaxima für die Datenauswertung herangezogen wird.

Es wird darauf hingewiesen, dass eine optische Entkopplung der beiden Abtastpunkte 297a und 297b dadurch erfolgen kann, dass anstelle eines Lichtwellenleiters 270 zwei Lichtwellenleiter verwendet werden, deren Enden jeweils eine Punktlichtquelle und einen Punktlichtdetektor darstellen. Um eine feste Zuordnung der jeweiligen Lichtwellenleiter zu den Abtastpunkten 297a bzw. 297b zu gewährleisten, ist es in diesem Fall erforderlich, dass auch die beiden Lichtwellenleiter zusammen mit dem Glasstab 237 um eine gemeinsame Drehachse rotieren.

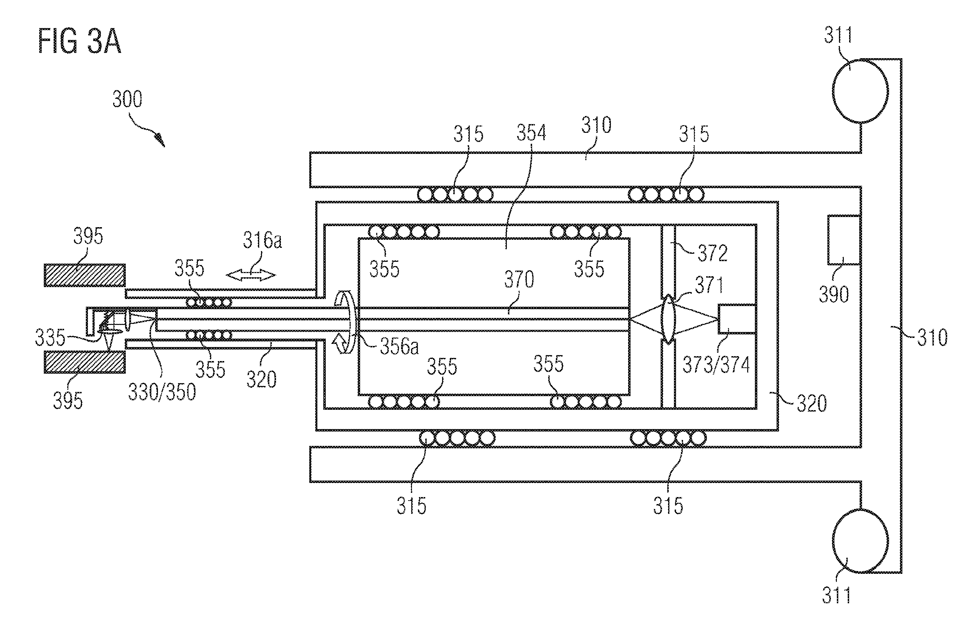

Figur 3a zeigt gemäß einem weiteren Ausführungsbeispiel der Erfindung eine konfokale Messvorrichtung 300, bei der anstelle einer chromatischen Abbildungsoptik eine radial verschiebbare Abbildungsoptik 335 vorgesehen ist. Die Messvorrichtung 300 umfasst ein Gehäuse 310, an welchem Adaptionselemente 311 vorgesehen sind, die in nicht dargestellter Weise eine Fixierung der Messvorrichtung 300 relativ zu einem Objekt 395, in welchem ein zu vermessender zylindrischer Hohlraum ausgebildet ist, ermöglichen. Die Adaptionselemente 311 können beispielsweise saugnapfartige mechanische Koppelelemente aufweisen, die eine feste, aber trotzdem lösbare Fixierung des Gehäuses 311 relativ zu dem Objekt 395 ermöglichen. Die Messvorrichtung 300 umfasst ferner einen Messkopf 320, welcher mittels mehrerer Linearlager 315 und einem nicht dargestellten Axialantrieb entlang einer Verschieberichtung 316a relativ zu dem Gehäuse 310 bewegt werden kann.

In dem Messkopf 320 ist ein zylindrischer Rotationskörper 354 mittels mehrerer Drehlager 355 drehbar gelagert, so dass der Rotationskörper 354 mittels eines nicht dargestellten Drehantriebes entlang einer Drehrichtung 356a drehbar ist. In dem Rotationskörper 354 ist ein Lichtwellenleiter 370 zentrisch eingegossen. Bei einer Drehung des Rotationskörpers 354 wird somit auch der Lichtwellenleiter 370 um dieselbe Drehachse gedreht, welche mit der Längsachse des Lichtwellenleiters 370 zusammenfällt. Der Lichtwellenleiter 370 dient als Lichttransportelement von einer Laserdiode 373 hin zu einer Punktlichtquelle 330 und als Lichttransportelement von einem Punktlichtdetektor 350 hin zu einer Photodiode 374.

Durch die zentrische Anordnung des Lichtwellenleiters 370 in dem Rotationskörper 354, welcher wiederum zentrisch zu dem Messkopf 320 angeordnet ist, bleibt bei einer Drehung des Rotationskörpers 354 der Faserkern des Lichtwellenleiters 370 stets in einer räumlich konstanten Position. Dies erlaubt somit auch während einer Rotation des Lichtwellenleiters 370 einen konstanten Lichttransport sowohl hin zu der Punktlichtquelle 330 und als auch weg von dem Punktlichtdetektor 350.

Es wird darauf hingewiesen, dass es bei dem Lichtwellenleiter 370 nicht darauf ankommt, dass der Lichtwellenleiter 370 auf seiner gesamten Länge zentrisch in dem Rotationskörper 345 angeordnet ist. Entscheidend ist lediglich, dass sich die beiden Enden des Lichtwellenleiter 370 auf der Rotationsachse des Lichtwellenleiters 370 befinden. Der räumliche Verlauf des Lichtwellenleiters 370 zwischen diesen beiden Enden ist solange nicht von Bedeutung, solange der Lichtwellenleiter 370 nicht so stark gekrümmt ist, dass für das in dem Lichtwellenleiter 370 geführte Licht zumindest teilweise keine Totalreflexion mehr auftritt.

Die Laserdiode 373 und die Photodiode 374 sind als gemeinsames optoelektronisches Modul ausgebildet. Auf diese Weise ist es nicht erforderlich, das sich objektseitig elektronische oder optoelektronische Komponenten der konfokalen Messvorrichtung 300 befinden, so dass die objektseitige Spitze des Messkopfes, welche während eines Messvorgangs in einen in dem Objekt 395 ausgebildeten und zu vermessenden zylindrischen Hohlraum eingeführt wird, in einer kompakten Bauweise realisiert werden kann.

Die Einkopplung von Beleuchtungslicht in den Lichtwellenleiter 370 sowie die Auskopplung von Messlicht aus dem Lichtwellenleiter 370 in die Photodiode 374 erfolgt über eine Abbildungsoptik 371, welche mittels einer Halterung 372 in dem Messkopf 320 fixiert ist. Das optoelektronische Modul mit der Laserdiode 373 und der Photodiode 374 ist mit einem Elektronikmodul 390 gekoppelt, welches sämtliche Funktionen der Steuer- der Regel- und der Messelektronik, welche für den Betrieb der konfokalen Messvorrichtung 300 erforderlich sind, durch entsprechende elektronische Schaltungen erfüllt.

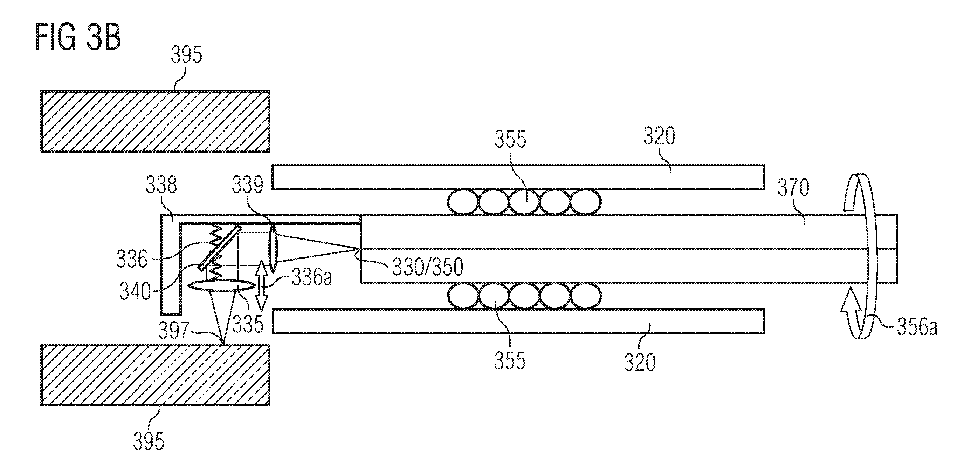

Figur 3b zeigt den vorderen, d. h. den objektseitigen Teil der konfokalen Messvorrichtung 300 in einer vergrößerten Abbildung. Das objektseitige Ende des Lichtwellenleiters 370, welcher entlang der Drehrichtung 356a um seine Mittelachse gedreht wird, dient als Punktlichtquelle 330.

Das von der Punktlichtquelle 330 ausgesandte Beleuchtungslicht wird von einer stationären Abbildungsoptik 339 auf einen drehbaren Reflektor 340 gelenkt. Die Abbildungsoptik 339 und der Reflektor 340 sind an einer Halterung 338 in nicht dargestellter Weise fixiert. Die Halterung 338 ist an dem Lichtwellenleiter 370 befestigt, so dass sich bei einer Drehung des Lichtwellenleiters 370 automatisch die Abbildungsoptik 339 und der Reflektor 340 um die Drehachse bzw. die Längsachse des Lichtwellenleiters 370 drehen.

Nach einer Reflexion an dem drehbaren Reflektor 340 trifft das Beleuchtungslicht auf eine Abbildungsoptik 335, welche entlang einer radialen Verschieberichtung 336a verschiebbar gelagert ist. Die Abbildungsoptik 335 ist von einer Feder 336 gehalten, welche sich zwischen der Halterung 338 und der verschiebbar gelagerten Abbildungsoptik 335 befindet.

Bei einer Drehung der Abbildungsoptik 335 um die Drehachse, welche Drehung gemeinsam mit einer Drehung des Lichtwellenleiters 370 erfolgt, wirkt somit auf die Abbildungsoptik 335 eine Zentrifugalkraft, die mit dem Quadrat der Winkelgeschwindigkeit der Drehbewegung ansteigt. Durch die Federkraft der Feder 336, welche eine Spiralfeder, eine Blattfeder oder ein beliebiges anderes Federelement bzw. elastisches Element sein kann, entsteht ein Kräftegleichgewicht, welches bei stationären Bedingungen, d. h. nach einem Einschwingverhalten zu einer festen radialen Position der Abbildungsoptik 335 führt. Somit kann über die Winkelgeschwindigkeit die jeweilige radiale Position der Abbildungsoptik 335 eingestellt werden. Die Position hängt dabei auch noch von konstanten Parametern wie der Masse der Optik, der Federkonstante der Feder 336 und der Nulllage der Feder 336 ab. Auf diese Weise kann durch eine Einstellung der Winkelgeschwindigkeit die Lage des Fokusses 397 des Beleuchtungslichtes eingestellt werden.

Entsprechend dem konfokalen Prinzip ist die auf den Punktlichtdetektor 335 bzw. in den Lichtwellenleiter 370 eingekoppelte Intensität an Messlicht dann maximal, wenn der Fokus 337 genau auf die innere Oberfläche des in dem Objekt 395 ausgebildeten Hohlraums trifft. Somit lässt sich durch eine Auswertung des von der Photodiode 374 gemessenen Messsignals abhängig von der Winkelgeschwindigkeit der rotierenden Abbildungsoptik 335 der Durchmesser des in dem Objekt 395 ausgebildeten zylindrischen Hohlraums bestimmen.

Um die gesamte Innenwand des in dem Objekt 395 ausgebildeten Hohlraums zu vermessen, ist zusätzlich zu der Rotationsbewegung der objektseitigen Komponenten der konfokalen Messvorrichtung 300 auch noch eine axiale Verschiebung dieser Komponenten entlang der Verschieberichtung 316a erforderlich.

Eine geeignete Messsequenz zur vollständigen Vermessung des zylindrischen Hohlraums besteht beispielsweise darin, dass zunächst eine konstante Rotationsgeschwindigkeit und damit eine feste Fokuslage eingestellt wird. Es folgt eine kontinuierliche axiale Verschiebung, so dass der gesamte Innenraum bei einer ersten Fokuslage des Beleuchtungslichts vermessen wird. Danach erfolgt in weiteren Messschritten eine schrittweise Erhöhung der Rotationsgeschwindigkeit, wobei bei jeder eingestellten Rotationsgeschwindigkeit erneut der objektseitige Teil der Messvorrichtung 300 axial verschoben wird. Auf diese Weise wird nacheinander der zylindrische Hohlraum in dem Objekt 395 mit einer Vielzahl von unterschiedlichen Fokuslagen vermessen, so dass durch eine geeignete Auswertung sämtlicher gesammelter Messsignale die Geometrie des zylindrischen Hohlraums vollständig erfasst werden kann.

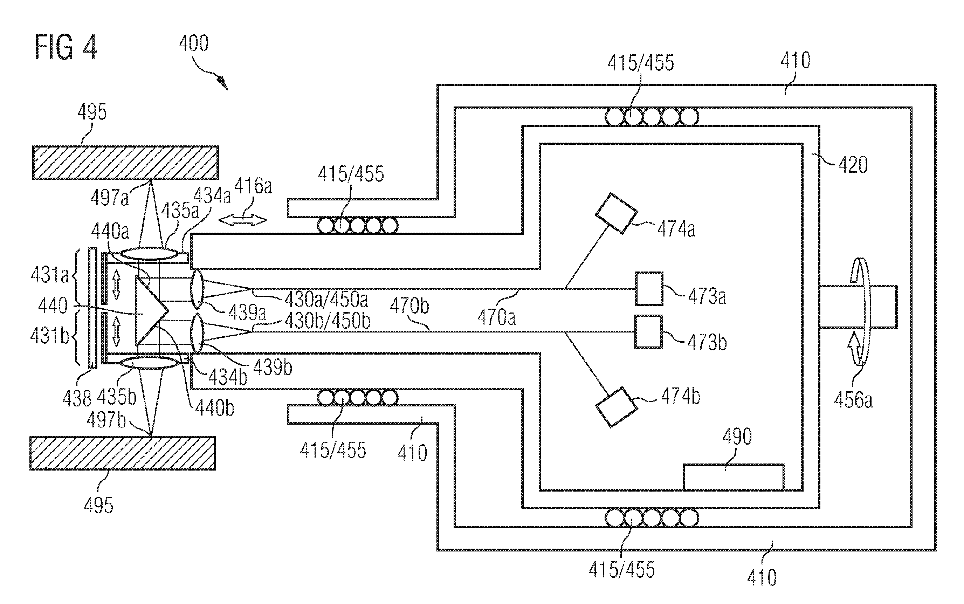

Figur 4 zeigt eine konfokale Messvorrichtung 400, bei der ein zylindrischer Hohlraum eines Objekts 495 an zwei voneinander unabhängigen Abtastpunkt gleichzeitig vermessen werden kann. Die Messvorrichtung 400 umfasst ein Gehäuse 410, in welchem eine Vielzahl von Lagern ausgebildet sind. Die Lager umfassen sowohl Linearlager 415 als auch Drehlager 455, so dass sowohl eine axiale Verschiebung eines Messkopfes 420 entlang einer Verschieberichtung 416a als auch eine Drehung des Messkopfes 420 entlang einer Drehrichtung 456a möglich ist. Sowohl die Drehung als auch die axiale Verschiebung des Messkopfes 420 erfolgen mittels nicht dargestellter Antriebe.

In dem Messkopf 420 sind zwei voneinander unabhängige konfokale Abstandssensoren aufgebaut, welche jeweils eine Punktlichtquelle 430a bzw. 430b sowie einen Punktlichtdetektor 450a bzw. 450b aufweisen. Die Punktlichtquelle 430a sowie der Punktlichtdetektor 450a sind durch das Ende eines Lichtwellenleiters 470a realisiert. In analoger Weise sind auch die Punktlichtquelle 430b sowie der Punktlichtdetektor 450b durch das Ende eines Lichtwellenleiters 470b realisiert. Um die als Punktlichtquellen bzw. als Punktlichtdetektor wirkenden objektseitigen Enden der Lichtquellenleiter 470a bzw. 470b jeweils sowohl mit einem optischen Sendeelement als auch mit einem optischen Empfangselement zu koppeln, sind die dem objektseitigen Ende gegenüberliegenden Ende des Lichtwellenleiters 470a bzw. 470b jeweils in zwei Teilenden aufgespalten.

Dabei ist jeweils eine erstes Teilende mit einer Laserdiode 473a bzw. 473b und ein zweites Teilende mit einer Photodiode 474a bzw. 474b gekoppelt. Auf diese Weise dienen die beiden Lichtwellenleiter 470a und 470b auch als Lichttransportmittel, um die beiden Punktlichtquellen 430a und 430b bzw. die beiden Punktlichtdetektoren 450a bzw. 450b möglichst nahe an den zu vermessenden Innenraum heranzuführen. Auf diese Weise kann das Beleuchtungslicht mit einer großen numerischen Apertur auf die zu vermessende Oberfläche bzw. die zu vermessende Seitenwand gelenkt werden, so dass eine hohe Auflösung bei der Abstandmessung erreicht werden kann.

Das von der Punktlichtquelle 430a ausgesandte Beleuchtungslicht trifft auf eine stationäre Abbildungsoptik 439a, welche das Beleuchtungslicht auf eine reflektierende Seite 444a eines Prismas 440 lenkt. Die als Reflektor wirkende Seite 440a des Prismas ist um einen Winkel von 45° gegenüber der optischen Achse des Beleuchtungslichts geneigt. Dadurch wird das Beleuchtungslicht in einem Winkel von 90° reflektiert und trifft auf eine radial verschiebbar gelagerte Abbildungsoptik 435a. Die Abbildungsoptik 435a ist an einer Halterung 434a befestigt, welche mit einem Radialantrieb 431a gekoppelt ist. Der Radialantrieb 431a weist zugleich ein Wegmesssystem auf, so dass die aktuelle Verschiebeposition genau bestimmbar ist. Durch eine radiale Verschiebung der Abbildungsoptik 435a kann die Fokuslage 497a des Beleuchtungslichts variiert werden. Entsprechend dem konfokalen Abbildungsprinzip ist die Intensität des von der zylindrischen Innenwand des in dem Objekt 495 ausgebildeten Hohlraums zumindest teilweise zurück gestreute Intensität genau dann besonders hoch, wenn der Fokus 497a genau auf die Innenfläche des zylindrischen Hohlraums trifft.

In entsprechender Weise wird das Beleuchtungslicht, welches von der Punktlichtquelle 430b emittiert wird, von einer Abbildungsoptik 439b auf eine reflektierende Seite 440b des Prismas 440 gelenkt. Nach einer Reflexion des Beleuchtungslichts an der ebenfalls um 45° gegenüber der optischen Achse des Beleuchtungslichtes geneigten reflektierenden Seite 440b des Prismas 440 wird das Beleuchtungslicht der Punktlichtquelle 430b dann diametral zu dem von der Punktlichtquelle 430a emittierten Beleuchtungslicht auf die Innenwand des zu vermessenden zylindrischen Hohlraums gerichtet.

Zur Variation der Fokuslage 497b ist in entsprechender Weise eine radial verschiebbare Abbildungsoptik 435b vorgesehen. Die Abbildungsoptik 435b ist an einer Halterung 434b befestigt, die ebenfalls mit einem Radialantrieb 431b fest verbunden ist. Auch der Radialantrieb 431b weist ein integriertes Wegmesssystem auf. Auf diese Weise kann auch die Fokuslage 497b durch eine entsprechende Ansteuerung des Radialantriebs 431b variiert werden. Durch eine Messung der zumindest teilweise zurück gestreuten und in den Lichtwellenleiter 474b eingekoppelten Lichtintensität, kann an dem jeweiligen Abtastpunkt der Abstand der zylindrischen Oberfläche 497 von der nicht dargestellten Drehachse bestimmt werden.

Die Vermessung der vollständigen Innenwand des zylindrischen Hohlraums erfolgt ebenso durch eine Kombination einer Drehbewegung des Messkopfes 420 entlang einer Drehrichtung 456a mit einer Verschiebung des Messkopfes 420 entlang der Verschieberichtung 416a.

Ferner ist in dem Messkopf 420 ein Elektronikmodul 490 vorgesehen, welches (a) eine Vielzahl von elektronischen Baugruppen aufweist, die sowohl die radiale Verschiebung der Abbildungsoptik 435a und 435b steuern bzw. regeln, welche (b) die Drehung und die Verschiebung des Messkopfes 420 relativ zu dem Gehäuse 410 steuern bzw. regeln und welche (c) die optoelektronischen Komponenten, die gemäß dem hier dargestellten Ausführungsbeispiel in dem rotierenden Messkopf 420 angeordnet sind, mit Strom versorgen und deren Messsignale entsprechend auswerten. Selbstverständlich können diese Funktionalitäten auch mittels Software vollständig oder zumindest teilweise realisiert werden.

Es wird darauf hingewiesen, dass die Halterung 438 in nicht dargstellter Weise mit dem Messkopf 420 verbunden ist. Damit drehen sich bei einer Rotation des Messkopfes nicht nur die innerhalb des Messkopfes 420 angeordneten optoelektronischen Komponenten, die beiden Lichtwellenleiter 470a und 470b und die beiden Abbildungsoptiken 439a und 439b um die Drehachse. In gleicher Weise drehen sich auch die direkt oder indirekt an der Halterung 438 befestigten Komponenten um die nicht dargestellte Drehachse. Wie aus Figur 4 ersichtlich, sind dies das Prisma 440 und die beiden radial verschiebbaren Abbildungsoptiken 435a und 435b.

Zur vollständigen Vermessung des zylindrischen Hohlraums können beispielsweise die beiden folgenden Abtastsequenzen verwendet werden, wobei die Messvorrichtung 400 bevorzugt jeweils mit einer konstanten Rotationsgeschwindigkeit betrieben wird:
a) Durch eine kontinuierliche radiale Verschiebung der Optiken 435a und 435b erfolgt ein Abtasten des zu vermessenden Innenraums auf einer die Drehachse umlaufenden Linie. Dieses Abtasten wird bei verschiedenen axialen Verschiebepositionen des Messkopfes 420 relativ zu dem Gehäuse 410 durchgeführt. Dadurch wird der zu vermessende Hohlraum in unterschiedlichen Tiefen an die Drehachse umlaufenden Linien vollständig vermessen. Die radiale Verschiebung der Optiken 435a und 435b erfolgt bevorzugt in einer kontinuierlichen Art und Weise, das Verschieben des Messkopfes 420 in axialer Richtung erfolgt bevorzugt in diskreten Schritten.
b) Die Fokuslage wird durch eine schrittweise Verschiebung der Abbildungsoptiken 435a und 435b in radialer Richtung variiert. Für jede radiale Verschiebeposition der Abbildungsoptiken 435a und 435b wird der Messkopf 420 kontinuierlich entlang des gesamten axialen Messbereichs verschoben.

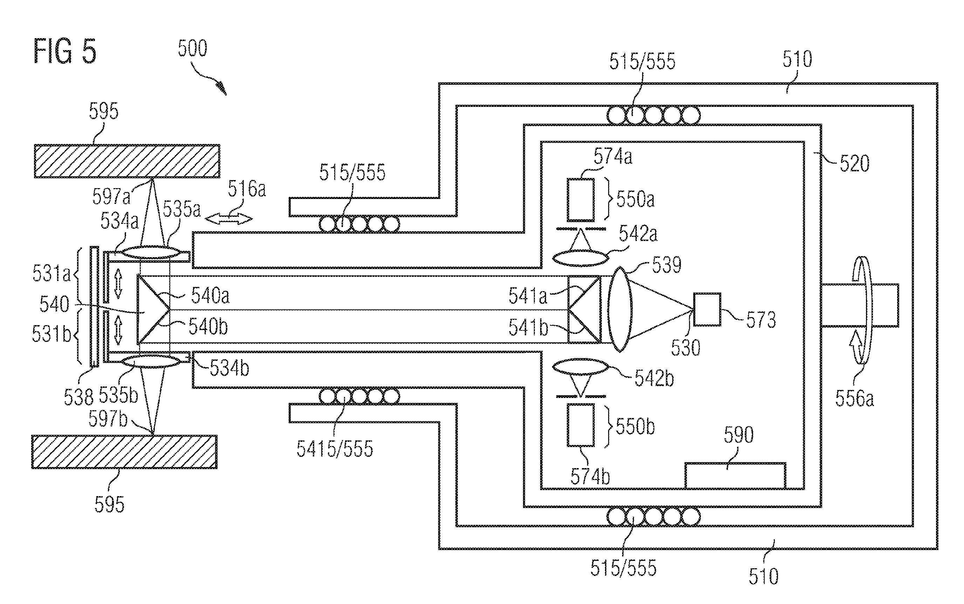

Figur 5 zeigt eine konfokale Messvorrichtung 500 gemäß einem weiteren Ausführungsbeispiel der Erfindung. Die konfokale Messvorrichtung 500 unterscheidet sich von der in Figur 4 dargestellten Messvorrichtung 400 lediglich dadurch, dass das Beleuchtungslicht auf eine modifizierte Art und Weise auf ein Prisma 540 gelenkt wird, welches zwei reflektierende Seiten 540a und 540b aufweist. Um Wiederholungen zu vermeiden, werden im folgenden nur noch diejenigen Komponenten der konfokalen Messvorrichtung 500 beschrieben, welche sich von den Komponenten der Messvorrichtung 400 hinsichtlich ihrer Struktur oder ihrer Funktion unterscheiden.

Die konfokale Messvorrichtung 500 weist einen Messkopf 520 auf, in dem lediglich eine Punktlichtquelle 530 vorgesehen ist. Die Punktlichtquelle 530 wird mittels einer Laserdiode 573 realisiert, welche gegebenenfalls hinter einer Lochblende (nicht dargestellt) angeordnet sein kann. Das von der Punktlichtquelle 530 emittierte Beleuchtungslicht trifft auf eine Abbildungsoptik 539, welche in dem Messkopf 520 stationär angeordnet ist. Die Abbildungsoptik 539 transformiert das von der Punktlichtquelle 530 ausgesandte divergierende Beleuchtungslicht in ein im wesentlichen paralleles Strahlenbündel, welches auf zwei Strahlteiler 541a und 541b trifft. Nach einer Transmission durch die Strahlteiler 541a bzw. 541b trifft das Beleuchtungslicht in gleicher Weise wie bei der in Figur 4 dargestellten Messvorrichtung 400 als paralleles Strahlenbündel auf das Prisma 540, von dem es in diametral entgegen gesetzte Richtungen gelenkt wird.

Das von der zylindrischen Innenwand des Objekts 595 zumindest teilweise zurück gestreute Licht wird in entsprechender Weise (siehe Figur 4) in den Strahlengang zwischen dem Strahlengang 540 und den beiden Strahlteilern 541a und 541b als Messlicht eingekoppelt. Nach einer Reflexion an dem Strahlteiler 541a bzw. 541b trifft das Beleuchtungslicht auf eine ebenfalls im Messkopf 520 stationär angeordnete Abbildungsoptik 542a bzw. 542b, welche das Messlicht auf einen Punktlichtdetektor 550a bzw. 550b lenkt. Der Punktlichtdetektor 550a bzw. 550b ist durch eine Photodiode 574a bzw. 574b mit einer davor angeordneten Lochblende realisiert.

Die Ansteuerung bzw. die Regelung der konfokalen Messvorrichtung sowie die Auswertung der von den Photodioden 474a und 474b aufgenommenen Messsignale erfolgt ebenso wie bei der in Figur 4 dargestellten Messvorrichtung 400 in einem Elektronikmodul 590.

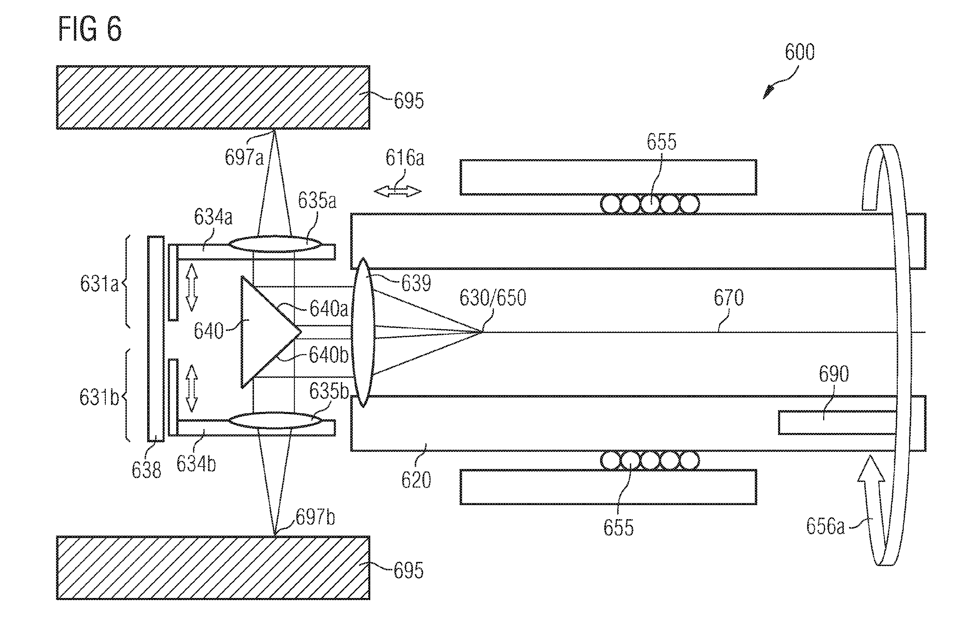

Figur 6 zeigt den vorderen, objektseitigen Teil einer weiteren Ausführungsform der Erfindung in Form einer konfokalen Messvorrichtung 600, welche sich von der in Figur 5 dargestellten Messvorrichtung 500 dadurch unterscheidet, dass die Punktlichtquelle 630 mittels eines Lichtwellenleiters 670 innerhalb des Messkopfes 620 näher an das objektseitige Ende der Messvorrichtung 600 herangeführt ist. Das von der Punktlichtquelle 630 ausgesandte Beleuchtungslicht wird mittels einer stationär im Messkopf angeordneten Abbildungsoptik 639 in ein paralleles Lichtbündel transformiert. Das parallele Lichtbündel trifft zentrisch auf ein Prisma 640, so dass das Beleuchtungslicht durch eine Reflexion an reflektierenden Seiten 640a bzw. 640b des Prismas 640 in diametral entgegengesetzte Richtungen gelenkt wird. Die Variation der Fokuslage sowie die Ansteuerung von sämtlichen mechanischen, elektronischen und optoelektronischen Komponenten der konfokalen Messvorrichtung 600 durch ein Elektronikmodul 690 erfolgt in gleicher Weise wie bei den zuvor anhand von Figur 4 und Figur 5 erläuterten Ausführungsformen.

Die beschriebene Ausführungsform hat den Vorteil, dass in dem objektseitigen Teil der konfokalen Messvorrichtung 300, welches den eigentlichen Scankopf darstellt, ein Minimum an elektrischen und mechanischen Stellgliedern eingesetzt wird. Dies ermöglicht die Herstellung eines miniaturisierten Messkopfes mit extrem geringen Abmessungen, so dass zumindest die objektseitige Spitze des Messkopfes auch in kleinere zylindrische Hohlräume einbringbar ist.

Eine besonders kompakte Bauform kann dann realisiert werden, wenn die optischen Komponenten der Messvorrichtung, welche sich insbesondere in dem objektseitigen Teil des Messkopfes befinden, optimal an einen bestimmten zu vermessenden Lochdurchmesser angepasst werden. Dies bedeutet, dass zur Vermessung von Löchern mit unterschiedlich großem Lochdurchmesser jeweils ein an den zu vermessenden Hohlraum angepasster Messkopf eingesetzt werden sollte.

Es wird darauf hingewiesen, dass die hier beschriebenen Ausführungsformen lediglich ein beschränkte Auswahl an möglichen Ausführungsvarianten der Erfindung darstellen. So ist es möglich, die Merkmale einzelner Ausführungsformen in geeigneter Weise miteinander zu kombinieren, so dass für den Fachmann mit den hier expliziten Ausführungsvarianten eine Vielzahl von verschiedenen Ausführungsformen als offensichtlich offenbart anzusehen sind.

### Zusammenfassend bleibt festzustellen:

Es wird eine optische Messvorrichtung zum Vermessen der Innenwand eines in einem Objekt ausgebildeten Hohlraums beschrieben. Die Messvorrichtung umfasst einen konfokalen Abstandssensor, bei dem der Strahlengang des Beleuchtungs- und des Messlichts mittels eines drehbar gelagerten Reflektors umgelenkt wird. Auf diese Weise kann durch ein Einführen zumindest eines objektseitigen Endes der Messvorrichtung in den zu vermessenden Hohlraum eine umlaufende Seitenwand des Hohlraums vermessen werden. Der Reflektor wird um eine Drehachse rotiert, welche bevorzugt mit der Längsachse der optischen Messvorrichtung zusammenfällt. Auf diese Weise erfolgt ein Abtasten der Innenwand entlang einer die Drehachse umlaufenden Linie.

Die optische Messvorrichtung kann auch mit zwei konfokalen Abstandssensoren ausgestattet sein, wobei am objektseitigen Ende der Messvorrichtung eine mehrere optische Komponenten aufweisende Doppeloptik vorgesehen ist, mittels welcher die Beleuchtungsstrahlen der beiden konfokalen Abstandssensoren bevorzugt diametral nach außen auf einander gegenüberliegende Abtastpunkte an der Innenwand des zu vermessenden Hohlraums fokussiert werden. Durch die diametrale Anordnung können auch bei einer nicht zentrischen Einführung der Messvorrichtung in einen zylindrischen Hohlraum sowohl der Durchmesser als auch der Mittelpunkt des zylindrischen Hohlraums präzise ermittelt werden.

Die Höhenmessung der seitlichen Innenwand kann mittels eines chromatischen konfokalen Abstandssensors erfolgen, bei dem gleichzeitig mehrere Fokuslagen erzeugt werden, die jeweils von der Wellenlänge des Beleuchtungslichts abhängen. Durch eine Spektralanalyse des von einem Punktlichtdetektor erfassten, von der Innenwand des Objekts zumindest teilweise zurück gestreuten Lichts kann der Abstand zwischen dem jeweiligen Abtastpunkt auf der Innenwand und dem rotierenden Reflektor bestimmt werden.

Die Fokuslage kann auch durch eine Variation des Strahlengangs mittels einer Verschiebung von Reflektoren oder einer Verschiebung von Abbildungsoptiken erfolgen. Eine derartige Verschiebung kann passiv mittels einer Feder erfolgen, die von einer bei der Rotation entstehenden Zentrifugalkraft einer optischen Komponente gespannt wird. Die Verschiebung kann jedoch auch aktiv mittels einer mechanischen Verstelleinheit für die betreffende optische Komponente des Abstandssensors erfolgen. Bei einer mechanischen Verschiebung von zumindest einer optischen Komponente des Abstandssensors wird von einem punktförmigen Detektor des konfokalen Abstandssensors eine Maximalintensität genau dann gemessen, wenn das Beleuchtungslicht exakt auf die Oberfläche der Innenwand des Hohlraums fokussiert wird. Durch eine Auswertung der maximalen Intensität für jeden Abtastpunkt kann somit der Hohlraum mittels einer Vielzahl von dreidimensionalen Messungen vollständig vermessen werden.

### Bezugszeichenliste

- 100: konfokale Messvorrichtung (chromatisch)
- 110: Gehäuse
- 111: Adaptionselement
- 115: Linearlager
- 116: Axialantrieb
- 116a: Verschieberichtung
- 117: Axiales Wegmesssystem
- 118: Messfühler
- 119: Maßstab
- 120: Messkopf
- 130: Punktlichtquelle
- 135: Abbildungsoptik (Chromat)
- 137: Glasstab
- 137a: dicker Abschnitt des Glasstabes
- 137b: dünner Abschnitt des Glasstabes
- 138: Schutzmantel / Halterung
- 140: drehbarer Reflektor
- 150: Punktlichtdetektor
- 155: Drehlager
- 156: Drehantrieb
- 156a: Drehrichtung
- 157: Riemen
- 158a: LED
- 158b: Photodiode
- 159: Markierung
- 170: Lichtwellenleiter
- 173: optoelektronisches Modul / Weißlichtquelle
- 174: optoelektronisches Modul / spektral auflösender Lichtdetektor
- 175: Halterung
- 190: Elektronikmodul (Steuer-, Regel- u. Messelektronik)
- 195: Objekt (Ohrmuschel)
- 197: Fokus
- 200: konfokale Messvorrichtung (chromatisch)
- 216a: Verschieberichtung
- 220: Messkopf
- 230: Punktlichtquelle
- 235: Abbildungsoptik (Chromat)
- 237: Glasstab
- 237a: dicker Abschnitt des Glasstabes
- 237b: dünner Abschnitt des Glasstabes
- 238: Schutzmantel
- 240: Prisma
- 240a/b: drehbarer Reflektor
- 250: Punktlichtdetektor
- 255: Drehlager
- 256a: Drehrichtung
- 258a: LED
- 258b: Photodiode
- 259: Markierung
- 270: Lichtwellenleiter
- 295: Objekt (mit zylindrischen Hohlraum)
- 297a/b: Fokus

- 300: konfokale Messvorrichtung
- 310: Gehäuse
- 311: Adaptionselement
- 315: Linearlager
- 316a: Verschieberichtung
- 320: Messkopf
- 330: Punktlichtquelle
- 335: Abbildungsoptik (radial verschiebbar)
- 336: Feder
- 336a: radiale Verschieberichtung
- 338: Halterung
- 339: Abbildungsoptik (stationär)
- 340: drehbarer Reflektor
- 350: Punktlichtdetektor
- 354: Rotationskörper
- 355: Drehlager

- 356a: Drehrichtung
- 370: Lichtwellenleiter
- 371: Abbildungsoptik
- 372: Halterung
- 373: Laserdiode
- 374: Photodiode
- 390: Elektronikmodul (Steuer-, Regel- u. Messelektronik)
- 395: Objekt (mit zylindrischen Hohlraum)
- 397: Fokus

- 400: konfokale Messvorrichtung
- 410: Gehäuse
- 415: Linearlager
- 416a: Verschieberichtung
- 420: Messkopf
- 430a/b: Punktlichtquelle
- 431a/b: Radialantrieb mit Wegmesssystem
- 434a/b: Halterung
- 435a/b: Abbildungsoptik (verschiebbar)
- 438: Halterung
- 439a/b: Abbildungsoptik (stationär)
- 440: Prisma
- 440a/b: drehbarer Reflektor
- 450a/b: Punktlichtdetektor
- 455: Drehlager
- 456a: Drehrichtung
- 470a/b: Lichtwellenleiter
- 473a/b: Laserdiode
- 474a/b: Photodiode
- 490: Elektronikmodul (Steuer-, Regel- u. Messelektronik)
- 495: Objekt (mit zylindrischen Hohlraum)
- 497a/b: Fokus

- 500: konfokale Messvorrichtung
- 510: Gehäuse

- 515: Linearlager
- 516a: Verschieberichtung
- 520: Messkopf
- 530: Punktlichtquelle
- 531a/b: Radialantrieb mit Wegmesssystem
- 534a/b: Halterung
- 535a/b: Abbildungsoptik (verschiebbar)
- 538: Halterung
- 539: Abbildungsoptik (stationär im Messkopf)
- 540: Prisma
- 540a/b: drehbarer Reflektor
- 541a/b: drehbarer Strahlteiler
- 542a/b: Abbildungsoptik (stationär im Messkopf)
- 550a/b: Punktlichtdetektor (durch Lochblende realisiert)
- 555: Drehlager
- 556a: Drehrichtung
- 573: Laserdiode
- 574a/b: Photodiode
- 590: Elektronikmodul (Steuer-, Regel- u. Messelektronik)
- 595: Objekt (mit zylindrischen Hohlraum)
- 597a/b: Fokus

- 600: konfokale Messvorrichtung
- 616a: Verschieberichtung
- 620: Messkopf
- 630: Punktlichtquelle
- 631a/b: Radialantrieb mit Wegmesssystem
- 634a/b: Halterung
- 635a/b: Abbildungsoptik (verschiebbar)
- 638: Halterung
- 639: Abbildungsoptik (stationär im Messkopf)
- 640: Prisma
- 640a/b: drehbarer Reflektor
- 650: Punktlichtdetektor (durch Lochblende realisiert)
- 655: Drehlager
- 656a: Drehrichtung
- 670: Lichtwellenleiter

- 690: Elektronikmodul (Steuer-, Regel- u. Messelektronik)
- 695: Objekt (mit zylindrischen Hohlraum)
- 697a/b: Fokus

## Patentansprüche

1. Optische Messvorrichtung zum Vermessen der Innenwand eines in einem Objekt (195, 395) ausgebildeten Hohlraums nach dem konfokalen Abbildungsprinzip, insbesondere zur Vermessung eines zylindrischen Bohrlochs und/oder zur Vermessung des Ohrkanals eines menschlichen oder tierischen Lebewesens, aufweisend
• ein punktförmiges optisches Sendeelement (130, 330, 430a), eingerichtet zum Aussenden von Beleuchtungslicht,
• eine Abbildungsoptik (135, 335, 435a) zum Fokussieren des Beleuchtungslichts in einen Objektbereich,
• ein punktförmiges optisches Empfangselement (150, 350, 450a),
- welches in Bezug auf die Abbildungsoptik (135, 335, 435a) konfokal zu dem optischen Sendeelement (130, 330) angeordnet ist und
- welches zum Empfang von Messlicht eingerichtet ist, das von der Innenwand des zu vermessenden Hohlraums zumindest teilweise zurückgestreut und über die Abbildungsoptik (135, 335, 435a) auf das Empfangselement (150, 350, 450a) gelenkt wird,
• eine Auswerteeinheit (190, 390, 490),
- welche mit dem Empfangselement (150, 350, 450a) gekoppelt ist und
- welche derart eingerichtet ist, dass eine maximale Intensität an Messlicht, welches auf das Empfangselement (150, 350, 450a) trifft, erfassbar ist, und
• einen Reflektor (140, 340, 440a), welcher in dem Strahlengang des Beleuchtungslichts und des Messlichts angeordnet ist,
wobei
der Reflektor (140, 340, 440a) um eine Drehachse drehbar ist, so dass durch das Beleuchtungslicht die Innenwand des zu vermessenden Hohlraums entlang einer die Drehachse umlaufenden Linie abtastbar ist.

2. Optische Messvorrichtung nach Anspruch 1, zusätzlich aufweisend
• ein Gehäuse (110, 310, 410) und
• einen optischen Messkopf (120, 320, 420), welcher relativ zu dem Gehäuse (110, 310, 410) entlang einer Verschiebeachse verschiebbar gelagert ist,
wobei
das Sendeelement (130, 330, 430), die Abbildungsoptik (135, 335, 435a), das Empfangselement (150, 350, 450a) und der Reflektor (140, 340, 440a) dem optischen Messkopf (120, 320, 420) zugeordnet sind.

3. Optische Messvorrichtung nach Anspruch 2, zusätzlich aufweisend
einen Antrieb (116) zum axialen Verschieben des Messkopfes (120) relativ zu dem Gehäuse (110) und
ein Wegmesssystem (117) zum Erfassen der Verschiebeposition des Messkopfes (120) relativ zu dem Gehäuse (110).

4. Optische Messvorrichtung nach einem der Ansprüche 2 bis 3, bei der
das Gehäuse (110, 310) Adaptionselemente (111, 311) zum definierten Anlegen der optischen Messvorrichtung (100, 300) an das Objekt aufweist.

5. Optische Messvorrichtung nach einem der Ansprüche 1 bis 4, zusätzlich aufweisend
• einen Drehantrieb (156) zum Drehen des Reflektors (140) um die Drehachse und
• einen Drehwinkeldetektor (158a, 158b) zum Erfassen des aktuellen Drehwinkels des Reflektors (140).

6. Optische Messvorrichtung nach einem der Ansprüche 1 bis 5, bei der
das optische Sendeelement (130, 330, 430a) und/oder das optische Empfangselement (150, 350, 450a) durch das Ende eines Lichtwellenleiters (170, 370, 470a) realisiert ist.

7. Optische Messvorrichtung nach einem der Ansprüche 1 bis 6, bei der
• im Strahlengang zwischen Sendeelement (130) und Objektbereich ein dispersives optisches Element (135) angeordnet ist und bei der
• das Empfangselement (150) ein spektrales Auflösungsvermögen aufweist, so dass von der Auswerteeinheit (190) die maximale Intensität an Messlicht, die auf das Empfangselement (150) trifft, in Abhängigkeit von der Wellenlänge des Messlichts erfassbar ist.

8. Optische Messvorrichtung nach Anspruch 7, wobei
• die Abbildungsoptik (135) das dispersive optische Element ist und wobei
• zwischen Abbildungsoptik (135) und Reflektor (140) ein Lichttransportelement (137) vorgesehen ist, welches gemeinsam mit dem Reflektor (140) um die Drehachse drehbar ist.

9. Optische Messvorrichtung nach einem der Ansprüche 1 bis 6, zusätzlich aufweisend
ein Mittel (336, 431a, 431b) zur Variation der Fokuslage (397, 497a, 497b).

10. Optische Messvorrichtung nach Anspruch 9, bei der das Mittel zur Variation der Fokuslage
eine Lagerung umfasst, welche eine Verschiebung der Abbildungsoptik (335, 435a, 435b) entlang einer Verschieberichtung ermöglicht, wobei
die Verschieberichtung parallel zu der optischen Achse der Abbildungsoptik (335, 435a, 435b) verläuft.

11. Optische Messvorrichtung nach Anspruch 10, wobei
die Abbildungsoptik (335) zwischen Reflektor (340) und Objektbereich angeordnet und gemeinsam mit dem Reflektor (340) um die Drehachse drehbar ist, und wobei
die Abbildungsoptik (335) mittels eines Federelements (336) in einer in Bezug auf die Drehachse radialen Position gehalten wird, wobei die radiale Position unter dem Einfluss einer Zentrifugalkraft variierbar ist, die bei einer Drehung auf die Abbildungsoptik (335) wirkt.

12. Optische Messvorrichtung nach einem der Ansprüche 9 bis 10, bei der
das Mittel zur Variation der Fokuslage ein aktives Stellglied (431a, 431b) ist.

13. Optische Messvorrichtung nach Anspruch 12, bei der
das Mittel zur Variation der Fokuslage zusätzlich ein weiteres Wegmesssystem aufweist.

14. Optische Messvorrichtung nach einem der Ansprüche 1 bis 13, zusätzlich aufweisend
• ein weiteres punktförmiges optisches Sendeelement (430b), eingerichtet zum Aussenden von weiterem Beleuchtungslicht,
• eine weitere Abbildungsoptik (435b) zum Fokussieren des weiteren Beleuchtungslichts in einen weiteren Objektbereich,
• ein weiteres punktförmiges optisches Empfangselement (450b),
- welches in Bezug auf die weitere Abbildungsoptik (435b) konfokal zu dem weiteren optischen Sendeelement (430b) angeordnet ist,
- welches zum Empfang von weiterem Messlicht eingerichtet ist, das von der Innenwand des zu vermessenden Hohlraums zumindest teilweise zurückgestreut und über die weitere Abbildungsoptik (435b) auf das weitere Empfangselement (450b) gelenkt wird, und
- welches ebenfalls mit der Auswerteeinheit (490) gekoppelt ist, so dass eine maximale Intensität an weiterem Messlicht, die auf das weitere Empfangselement (450b) trifft, erfassbar ist, und
• einen weiteren Reflektor (440b), welcher in dem Strahlengang des weiteren Beleuchtungslichts und des weiteren Messlichts angeordnet ist,
wobei
der weitere Reflektor (440b) winklig zu dem Reflektor (440a) angeordnet und gemeinsam mit dem Reflektor (440a) um die Drehachse drehbar ist, so dass durch das weitere Beleuchtungslicht die Innenwand des zu vermessenden Hohlraums ebenfalls entlang einer die Drehachse umlaufenden Linie abtastbar ist, wobei das Beleuchtungslicht und das weitere Beleuchtungslicht in Bezug auf die Drehachse in unterschiedliche Richtungen an die Innenwand des Hohlraums gelenkt werden.

15. Optische Messvorrichtung nach Anspruch 14, bei der der Objektbereich und der weitere Objektbereich in Bezug auf die Drehachse diametral zueinander angeordnet sind.
